# EUROPEAN PATENT APPLICATION

(11) **EP 2 143 729 A1**
(43) Date of publication of application: **13.01.2010**
(21) Application number: 08012261.7
(22) Date of filing: 07.07.2008
(51) Int. Cl.: C07K 14/01, C12N 9/14

(54) **New enzymatic active enzyme against Clostridium**

(71) Applicant: Hyglos Invest GmbH, 82347 Bernried (DE)
(72) Inventor: Loessner, Martin, 8123 Ebmatingen (CH); Eichenseher, Fritz, 8050 Zürich (CH)
(74) Representative: Dehmel, Albrecht

(57) **Abstract**

The present invention refers to a polypeptide having the amino acid sequence as depicted in SEQ ID NO: 1, a variant or a fragment thereof. The present invention further refers to nucleic acids encoding said polypeptides. The present invention further refers to the use of the polypeptides or the fragments thereof as a therapeutic or diagnostic agent as well as for the contamination of food and feedstuff.

## Description

The present invention refers to a polypeptide having the amino acid sequence as depicted in SEQ ID NO:1, a variant or a fragment thereof. The present invention further refers to nucleic acids encoding said polypeptides. The present invention further refers to the use of the polypeptides or the fragments thereof as a therapeutic or diagnostic agent as well as for the contamination of food and feedstuff.

*Clostridium* is a heterogenic group of bacteria with more than 150 validly described species. Several clostridia have the capability to produce toxins and some of them are the most potent natural toxins known. Botulism, tetanus, enterotoxemic infections or necrotizing soft tissue infections are prominent examples of human diseases caused by bacteria of this genus. Beside negative impacts on farm- and domestic animals, some can cause defects in cheese during ripening and impair the milk processing industry.

*Clostridium perfringens* are Gram positive, anaerobic, spore-forming bacteria widely distributed in soil, sediments, food and the gastrointestinal tract of humans and animals. A large spectrum of diseases in humans and livestock animals is related to *C. perfringens*, including food poisoning, gas gangrene (clostridial myonecrosis), enteritis necroticans, and non-foodborne gastrointestinal infections. *C. perfringens* bacteriophage encoded endolysins that are responsible for virion release of their host cell can potentially be used for specific detection and control of these pathogenic bacteria.

The species produces at least 12 extracellular toxins. Some of them are considered as major lethal toxins. The strains are classified into 5 types A-E according to their ability to produce the toxins α, β, ε and

The α cpe gene encoding for the *C. perfringens* enterotoxin (CPE) can either be located on a plasmid or on the chromosome. Food poisoning Type A strains bear the chromosomal variant of cpe, whereas non food poisoning Type A strains have it plasmid encoded. The strains carrying α cpe on a plasmid seem to be responsible for a CPE associated human GI disease which is a non-food borne infection of the gastrointestinal tract.

The β toxin producing *C. perfringens* type C can cause another, unrelated and rarely reported disease, commonly known as "pigbel" occurring in New Guinea or "Darmbrand" ("fire-bowels"), a disease that occurred in Germany in the period after the Second World War. This segmental necrotizing enteritis syndrome affects the small intestine and has been associated with a mortality rate of 35-40 %.

Type A food-poisoning *C. perfringens* strains can be found in soil, water, food, dust, spices and the intestinal tract of humans and animals. These strains have been found in nearly all soil samples at levels of 10³ to 10⁴ CFU/g, whereas type B, C, D and E strains do not exist in soils, but are obligate parasites frequently found in domestic animals.

The heat sensitive Type A CPE is responsible for the food mediated illness. It has a molecular weight of 35 kDa and an isolelectric point of 4.3. The production of CPE is closely related to the late phase of sporulation and the toxin is released together with the endospores when the host cell lyses. Conditions that promote sporulation of the cells also promote the production of CPE.

Gas Gangrene or clostridial myonecrosis is an aggressive necrotizing soft tissue infection caused to about 80 % by C. *perfringens.* The disease is defined as acute invasion of healthy living muscle, undamaged by previous trauma or ischemia. *C. perfringens* Type A α toxin and to a lesser extent the perfringolysin O (0 toxin) are responsible for the pathogenesis. In most cases the traumatic gas gangrene occurs after deep penetrating injuries that damage blood supply. The histotoxicity of *C. perfringens* can lead to extensive wounds and if not treated, it can progress to systemic shocks, multi-organ failure and death.

*C. perfringens* is also responsible for a variety of veterinary diseases affecting farm- and domestic animals. The bacteria can be the causative agent of several enteric diseases of lambs and sheep, calves and cattle, goats, pigs, fouls and adult horses, domestic chickens, some species of captive wild birds and rabbits.

The presence of *C. perfringens* in the intestinal tract of an animal can result in necrotic enteritis, food poisoning and growth retardation, for example. In particular, the presence of *C. perfringens* in the intestinal tract of poultry, in particular broiler chickens, has been linked with various conditions, such as gut lesions and necrotic enteritis, and can result in a significant reduction in the growth of poultry. In addition, the widespread use of antibiotics in animal feed for growth promotion and disease prophylaxis is assumed to lead to aquired resistance to a series of therapeutically used antimicrobial agents such as tetracycline, bacitracin, erythromycin or cephalosporin.

Diarrhea accompanied by severe abdominal pain is the most common clinical symptom of the foodborne infection. Nausea sometimes occurs, fever and vomiting is unusual and death is uncommon, but possible in debilitated or institutionalized persons and especially affects elderly people. The incubation time after ingestion is 6-24 hours. Some cases have been reported were symptoms appeared only 2 hours after ingestion. It is assumed that pre-formed toxins or the consumption of already sporulated cells can lead to the appearance of symptoms briefly after ingestion. Normally the duration of the illness is short and symptoms disappear after a day or less. Elder or debilitated people sometimes need more time to convalesce.

Meat or meat products stored at too high temperatures are the most common source of *C. perfringens* food poisoning. Insufficient cooking also accounts for a high number of outbreaks. Seafood, dairy products, fruits and vegetables sometimes mediate the *C. perfringens* food poisoning, but for about 75 % of all outbreaks, meat or meat products are responsible. Contamination of meat by *C. perfringens* comes directly from slaughter animals or from a subsequent contamination from containers, dust or handlers. The infection dose of food mediated enterotoxin producing *C. perfringens* is assumed to be very high. About 108 vegetative cells seem to be necessary to cause diarrhea symptoms in humans. The bacterial spores are able to survive cooking temperatures and after the heating process, the competing flora is eliminated. They are able to multiply rapidly when the food cools down and reaches temperatures were germination of the spores and growth of vegetative cells is possible. The majority of outbreaks occur in restaurants and catering. There are only 3 % documented *C. perfringens* related food poisonings in private homes, but regarding the relatively mild course of the disease, the estimated number of unreported cases is thought to be higher. Hardly any outbreaks could be linked to commercially processed food; Hatheway, 1990, Clin. Microbiol. Rev. 3/1, 66-98; Rood & Cole, 1991, Microbiol. Rev. 55/4, 621-648; Songer, 1996, Clin. Microbiol. Rev. 9/2, 216-234.

The use of endolysins for diagnostics and to kill contaminating bacteria in food was first disclosed in GB 2,255,651. First therapeutic and prophylactic applications *in vivo* using mouse model systems were described by Nelson & Fischetti, (2001, Proc. Natl. Acad. Sci. USA 98, 4107-4112; Loeffler et al, 2001, Science 94, 2170-2172). A comprehensive review of the use of phage lytic enzymes for the control of pathogenic bacteria is given in Fischetti, 2006, BMC Oral Health 6, 16-19. A first lysine from a *C. perfringens* bacteriophage, namely phi3626 was disclosed in WO 03/066845.

However, there is still a great need for new substances acting as antimicrobials against *C. perfringens* as well as new methods for diagnosis of *C. perfringens.*

The problem is solved by the subject matter as disclosed in the claims.

To illustrate certain aspects of the invention, the following drawings are presented. They are not intended to restrict the invention in any way.

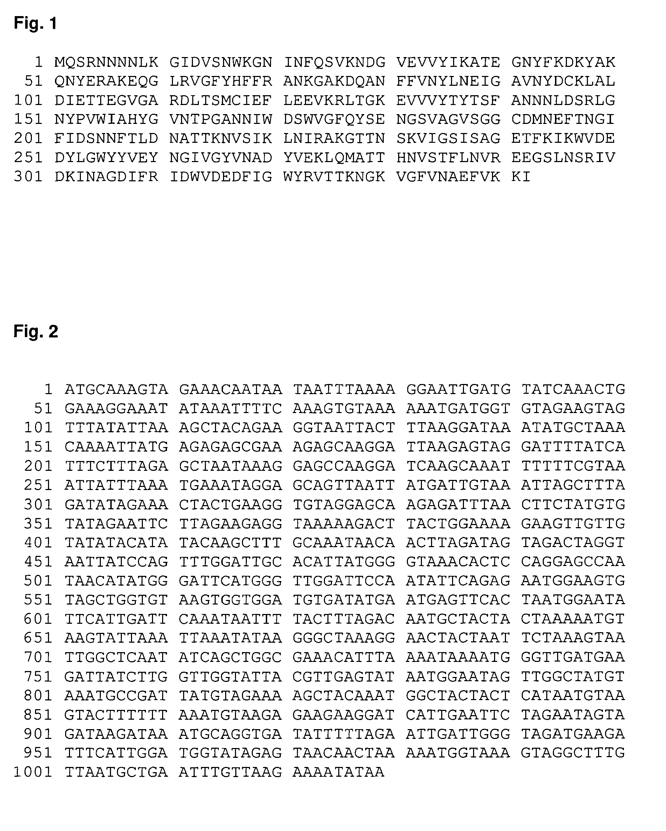

**Figure 1** shows an amino acid sequence (SEQ ID NO:1) of the lysin plyS9 according to the present invention.
**Figure 2** shows the nucleotide sequence (SEQ ID NO:2) encoding the lysin plyS9 according to the present invention.

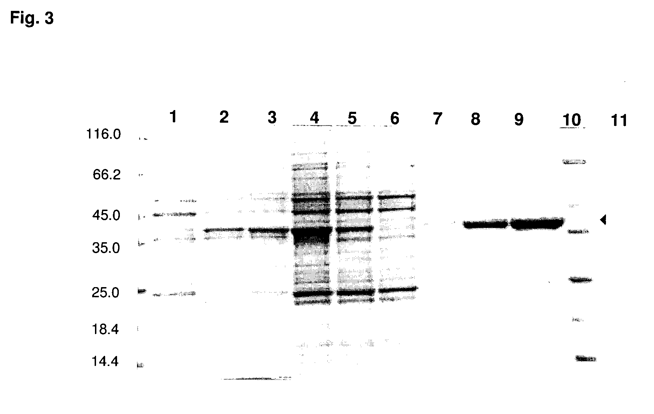

**Figure 3** is a Coomassie-stained SDS-PAGE showing the result of an analysis of plyS9 expression and purification. Lanes 1 and 11 pertain to size markers determined by the digits on the left hand side. Lanes 2, 3, 4 and 5 pertain to expression of the desired protein after 0h, 1h, 2h and 4h, respectively. Lane 6, 7, 8 and 9 pertain to the crude extract applied onto the column, the flow through, the washing fraction and the eluate of the purification process, respectively. Lane 10 pertains to the purified and concentrated protein in the presence of 500 mM NaCl and 50% glycerol. The arrowhead on the right hand side marks the position of purified plyS9.

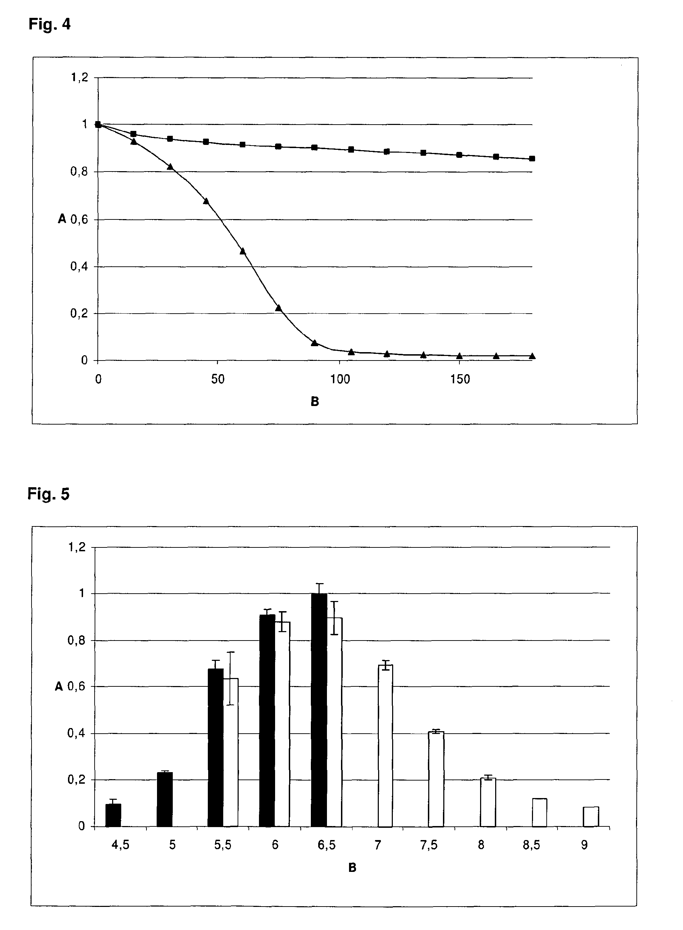

**Figure 4** is a graphic representation of a lysis curve of plyS9 against *C. perfringens* substrate cells under optimized conditions (6 µg plyS9; citrate/phosphate buffer pH 6.5 with 300 mM sodium chloride). "A" illustrates OD₆₀₀ and. "B" illustrates time [s]. The curves are standardized to an initial OD₆₀₀=1. Squares represent the control, triangles represent the plyS9 lysis curve.
**Figure 5** shows in a graphic representation the relative enzymatic activities (A) of plyS9 depending on pH (B) and buffering substance (black bars citrate/phosphate buffer and white bars Tris/phosphate buffer). Buffer composition was 25 mM citrate or Tris, 25 mM phosphoric acid, 120 mM NaCl; protein concentration was 0.5 µg plyS9.

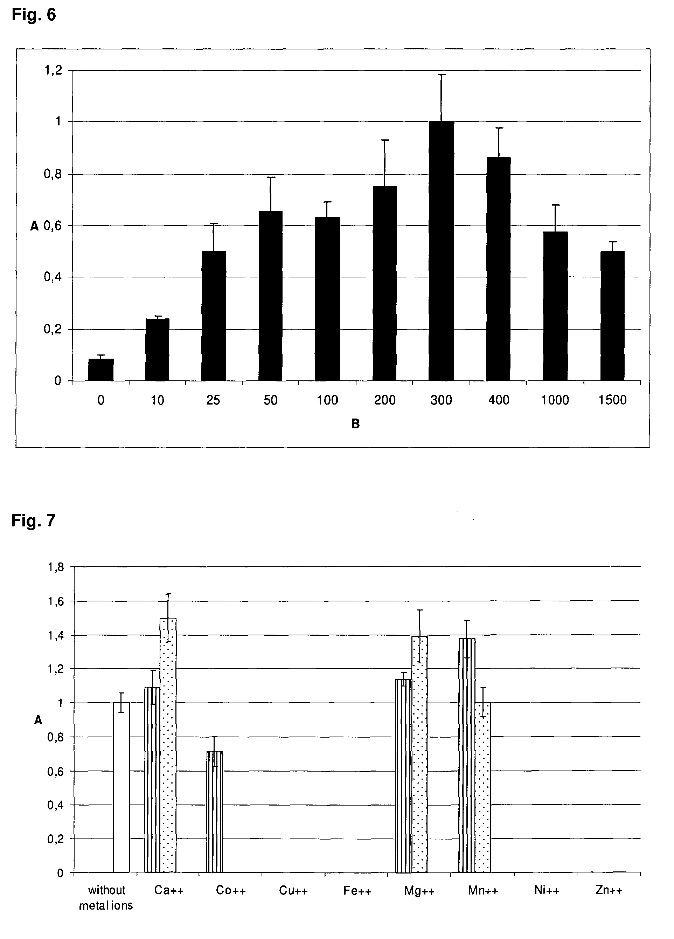

**Figure 6** shows in a graphic representation the relative enzymatic activities (A) of plyS9 depending on sodium chloride concentration (B). Buffer composition was 20 mM MOPS pH 7.0 and variing concentrations of NaCl [mM]; protein concentration was 0.5 µg plyS9).
**Figure 7** shows in a graphic representation the influence of divalent metal-ions on plyS9 activity (20 mM MOPS buffer pH 7.0, 300 mM Nacl; 0.5 µg plyS9). "A" represents relative enzymatic activity, "0" means without any metal ions. Bars filled with dots represent a metal ion concentration of 10 mM, bars filled with lines represent a metal ion concentration of 1 mM.

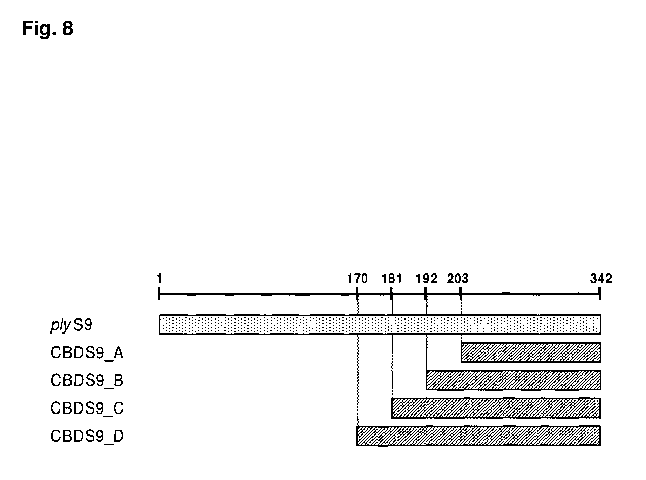

**Figure 8** is a schematic representation of plyS9 and specific length variants of the CBD region. The numbers represent the amino acid residues of the amino acid sequence as depicted in SEQ ID NO:1

The term "endolysin" as used herein refers to an enzyme which is suitable to hydrolyse bacterial cell walls. Endolysins from bacteriophages infecting Gram positive bacteria show a modular design. Mostly, the N-terminal domain is the enzymatically active domain (EAD), the C-terminal domain, which is designated as the cell wall binding domain (CBD) is responsible for substrate recognition. Both domains are connected by a short linker peptide. Endolysins comprise at least one EAD selected from the group of endopeptidase, N-acetyl-muramoyl-L-alanine-amidase, N-acetyl-muramidase and N-acetyl-glucosaminidase.

The term "domain" as used herein refers to a subunit of an endolysin which is ascribed a specific function and can also coincide with structural domains. The term domain is preferentially used to describe the antagonism between EAD which can be composed of more than one module and CBD domains.

The term "CBD" as used herein refers to the cell wall binding domain of an endolysin, which is often found at the C-terminus of the protein. CBD domains have no enzymatic activity in terms of hydrolyzing the cell wall, but often mediate binding of the endolysin to the bacterial cell wall.

The term "EAD" as used herein refers to the enzymatically active domain of an endolysin which is responsible for hydrolysis of the bacterial peptidoglycan. It contains at least one of the enzymatic activities of an endolysin selected from the group of endopeptidase, N-acetyl-muramoyl-L-alanine-amidase, N-acetyl-muramidase and N-acetyl-glucosaminidase

One aspect of the present invention refers to a polypeptide comprising the sequence of SEQ ID NO: as well as variants thereof. Said polypeptide as well as variants thereof is considered to be the polypeptide according to the present invention. All of them share the common feature to possess the activity of an endolysin and are thus capable to lyse *C. perfringens* bacteria. Said specificity for *C. perfringens* can be tested by a plurality of methods which are known in the art, e.g. by adding the polypeptide to a sample comprising one or more of said *C. perfringens* species and determining the change in turbidity after addition of the polypeptide.

In a preferred embodiment, a variant of the polypeptide according to the present invention exhibits single or multiple substitutions with regard to SEQ ID NO: 1.

Another aspect of the present invention refers to fragments of the polypeptide according to the present invention. The inventors of the present invention discovered that specific C-terminal fragments of plyS9 can bind to *C*. *perfringens.* Thus, a further preferred embodiment of the present invention relates to the CBD of plyS9. Preferred is a fragment of plyS9 comprising the amino acid residues 170 to 342, 181 to 342, 192 to 342, or 203 to 342 according to the amino acid sequence depicted in SEQ ID NO:1. Further preferred is a fragment of plyS9 beginning at an amino acid residue in the range of 170 and 203 and ending at the amino acid residue 342 according to the amino acid sequence depicted in SEQ ID NO: 1.

In a further preferred embodiment the variant of plyS9 comprises additional marker moieties such as biotin or tags such as HA-tag, His-tag, Strep-tag, Myc-tag, GST-tag, JS-tag or other tags known in the art.

It has to be understood that the above illustrated variants of plyS9 must not be considered as strictly separate embodiments but instead may be combined. For example, a variant of PlyS9 according to the present invention may comprise one or more amino acid residue substitutions as indicated above and a JS-tag with the proviso that such recombinant endolysin is still specific for *C. perfringens.* The person skilled in the art will readily realize which of said variant is suitable for his purposes and can always test such variant for activity against *C*. *perfringens* by routine methods in the art, for example by assaying the effect of lysis activity on the optical density of a *C. perfringens* containing solution.

The JS-tag derives from the biotin aczeptor domain of the α subunit of the *Klebsiella pneumoniae* oxalacetatedescarboxylase and comprises the consensus sequence MKM (K can be biotinylated), so that the polypeptide can be biotinylated *in vivo* by means of the enzyme biotin ligase. The JS-tag is a size reduced fragment of the entire α subunit of the *Klebsiella pneumoniae* oxalacetatedescarboxylase. A suitable minimal sequence of the JS-tag comprises 66 amino acid residues and is depicted in SEQ ID NO:14. However, derivatives of the 66 amino acid residues long JS-tag are suitable also and comprise sequences which are at least to 80, 90, 95, 98 % homolog to SEQ ID NO:14. Preferably, said derivatives are up to 80 amino acid residues long are at least to 80, 90, 95, 98 % homolog to SEQ ID NO:14 over a amino acid stretch of 66 amino acid residues.

A further aspect of the present invention relates to methods to detect *C. perfringens* in food, medical or veterinary samples, e. g. tissues of dead animals, as well as in animal feed and human food by means of the polypeptide, variant or fragment thereof according to the present invention.

The polypeptide or a variant thereof may be used to lyse *C. perfringens* in an assay prior to the detection. A common property of some assays is the need for release of nucleic acids (DNA or RNA) or biochemical components, e.g. ATP or intracellular enzyme activities from the bacterial cells to be determined.

However, the isolation of nucleic acid molecules enabled by the currently available techniques is characterised by significant disadvantages which are overcome by using the polypeptide or variant according to the present invention.

Preferably, the polypeptide or variant thereof is used in or prior to nucleic acid detection assay such as PCR, or other detection assays. In this respect, the polypeptide or variant thereof according to the present invention is used in or prior to nucleic acid molecule detection methods such as PCR to rapidly lyse *C. perfringens* cells, thereby releasing the genomic DNA or RNA. Once the cells have been lysed there is no need for an isolation and/or purification of the DNA or RNA for the amplification reaction.

In another aspect of the present invention the enzymatically active domain is substituted with a polypeptide being a marker. Thus, the cell wall binding domain or fragment thereof of the polypeptide PlyS9 is coupled to marker moieties such as biotin, Strep-Tag, fluorescence proteins such as Green fluorescence protein or other markers known in the art. Said CBD coupled to a marker moiety can be used in a method for rapid detection of *C. perfringens* e.g. in food.

Therefore, the present invention further provides a method of detecting a *Clostridium* bacterium, preferably *Clostridium perfringens*, in a sample, using the polypeptide, variant or CBD thereof according to the present invention.

In another aspect the present invention relates to a nucleic acid molecule comprising a sequence encoding the polypeptide, variant and fragment thereof of the present invention. It is apparent to a person skilled in the art that there may be many ways of constructing nucleic acid molecules encoding for the polypeptides, variants or fragments thereof according to the present invention, for example in view of the degenerate genetic code. A person skilled in the art with knowledge about the amino acid sequence of said polypeptides, variants or fragments thereof of the present invention is capable of choosing an adequate nucleotide sequence which suits his purposes most, for example in which the codon usage has been adapted to the codon usage of his desired expression host. A nucleic acid molecule comprising a sequence encoding for a polypeptide, variant or fragment thereof of the invention is considered to be the nucleic acid molecule or nucleic acid sequence, respectively, of the present invention.

In a preferred embodiment said nucleic acid molecule encodes for the polypeptide according to the present invention as depicted in SEQ ID NO:1. Preferably, said nucleic acid molecule comprises the nucleic acid sequence as depicted in SEQ ID NO:2.

In a preferred embodiment said nucleic acid molecule encodes for a fragment according to the present invention as depicted in SEQ ID NO:10-13. Preferably, said nucleic acid molecule comprises the nucleic acid sequence as depicted in SEQ ID NO:11.

In a further aspect the present invention relates to a vector comprising a nucleotide sequence of the invention. Preferably, said vector provides for the expression of said polypeptide, variant or fragment thereof of the invention in a suitable host cell. Said host cell may be selected due to mere biotechnological reasons, e.g. yield, solubility, costs, etc. but may be also selected from a medical point of view, e.g. a non-pathological bacteria or yeast, human cells, if said cells are to be administered to a subject. Said vector may provide for the constitutive or inducible expression of said polypeptides according to the present invention.

In addition to the specific nucleotide and amino acid sequences mentioned herein and amino acid sequences derivable from said nucleotide sequences, the present invention also encompasses variants, homologues and derivatives thereof.

Here, the term "homology" can be equated with "identity". In the present context, a homologous sequence is taken to include an amino acid sequence or a nucleotide sequence which may be at least 75%, 85% or 90% identical, preferably at least 95% or 98% identical. In particular, homology should typically be considered with respect to those regions of the sequence known to be essential for an activity. Although homology can also be considered in terms of similarity (i.e. amino acid residues having similar chemical properties/functions), in the context of the present invention it is preferred to express homology in terms of sequence identity.

In a preferred aspect, the variant, homologue or derivative is an amino acid sequence which is at least 75%, 85% or 90%, preferably at least 95% or 98% identical, with the amino acid sequence shown in SEQ ID NO: 1.

In a preferred aspect, the variant, homologue or derivative is a nucleotide sequence which is at least 75%, 85% or 90%, preferably at least 95% or 98% identical, with the nucleotide sequence shown in SEQ ID NO:2.

In a further aspect, the variant, homologue or derivative is an amino acid sequence which is at least 75%, 85% or 90%, preferably at least 95% or 98% identical, with the cell wall binding domain of the amino acid sequence shown in SEQ ID NO: 10-13. Preferably, the derivative comprises the amino acid sequence shown in SEQ ID NO: 11.

The present invention further relates to a medicament comprising a polypeptide or variant thereof according to the present invention. The present invention further relates to a pharmaceutical composition comprising the polypeptide, variant or fragment thereof according to the present invention.

In a further aspect of the present invention the above mentioned polypeptide, variant or fragment thereof are employed in a method for the treatment or prophylaxis of *Clostridium* infections in a subject, in particular for the treatment or prophylaxis of infections by *C. perfringens* or *C. tyrobutyricum.* Said subject may be a human subject or an animal, in particular animals used in livestock farming and/or dairy farming such as pigs, cows, sheeps, horses, poultries, goats, captive wild birds and rabbits Said method of treatment encompasses the application of said polypeptides of the present invention to the site of infection or site to be prophylactically treated against infection in a sufficient amount.

In particular, said method of treatment may be for the treatment or prophylaxis of infections of *Clostridium,* in particular by *C. perfringens,* of soft tissues and the gastrointestinal tract.

In a further preferred embodiment a polypeptide according to the present invention is employed in a method for the treatment or prophylaxis of myonecrosis, in particular of clostridial myonecrosis (Gas gangrene), Enteritis necroticans, in particular Enteritis necroticans caused by *C. perfringens* ("pig-bel" or "Darmbrand"), of infections of the gastrointestinal tract, in particular necrotizing enterocolitis of infants, enterotoxin-induced diarrhea or food borne illness caused by *C. perfringens*

In a further preferred embodiment a polypeptide according to the present invention is used in a method of treatment or prophylaxis of *Clostridium,* preferably of *C. perfringens* infections in various animals, in particular, enterotoxemia in sheep, goat, calves, cattle, horses and other animal species. In particular a polypeptide of the present application is suitable for use in methods of treatment or prophylaxis of dysentery and pulpy kidney disease in lamb or necrotic enteritis in poultry caused by *Clostridium,* in particular caused by *C. perfringens.*

In a particularly preferred embodiment a polypeptide or the variants thereof of the present invention is used for medical treatment, if the infection to be treated or prevented is caused by resistant or multiresistant *Clostridium* strains, in particular by *C. perfringens.* Furthermore, a polypeptide or the variants thereof of the present invention can be used in methods of treatment by administering them in combination with conventional antibacterial agents, such as antibiotics or other endolysins.

The dosage and route of administration used in a method of treatment or prophylaxis according to the present invention depends on the specific disease and/or site of infection to be treated. The route of administration may be for example in particular embodiments for oral, topical, parenteral, intravenous, rectal or any other route of administration.

For application of a polypeptide of the present invention to a site of infection or site endangered to be infected a polypeptide of the present invention may be formulated in such manner that the polypeptide is protected from environmental influences such as proteases, oxidation or immune response.

Therefore, a polypeptide of the present invention may be formulated as capsule, dragee, pill, suppository, injectable solution or any other medical reasonable galenic formulation. In some embodiments these galenic formulation may comprise suitable carriers, stabilizers, flavourings, buffers or other suitable reagents.

For example, for topical application a polypeptide of the present invention may be administered by way of a lotion or plaster.

For treatment of the intestine, for example in necrotizing enterocolitis or enterotoxin- induced diarrhea, suppository formulation can be envisioned. Alternatively, oral administration may be considered. In this case, the polypeptide of the present invention has to be protected from the harsh digestive environment until the site of infection is reached. This can be accomplished for example by using bacteria as carrier, which survive the initial steps of digestion in the stomach and which secret later on a polypeptide of the present invention into the intestinal environment.

All medical applications rely on the effect of the polypeptides of the present invention to lyse specifically and immediately *C. perfringens* bacteria when encountered. This has an immediate impact on the health status of the treated subject by providing a reduction in pathogenic bacteria and bacterial load and simultaneously relieves the immune system. Thus, the mayor task a person skilled in the art faces is to formulate the polypeptides of the present invention accurately for the respective disease to be treated. For this purpose usually the same galenic formulation as employed for conventional medicaments for these applications can be used.

In a further aspect of the present invention the above mentioned polypeptide or variant is a component of a pharmaceutical composition, which optionally comprises a carrier substance.

In an even further aspect the polypeptide or variant is part of a cosmetics or disinfectant composition. As mentioned above, several *Clostridium* species can cause irritations on environmentally exposed surfaces of the patient's body such as the skin. In order to prevent such irritations or in order to eliminate minor manifestations of said *Clostridium* pathogens, special cosmetic preparations may be employed, which comprise sufficient amounts of polypeptides of the present invention in order to lyse already existing or freshly settling *Clostridium.*

In a further aspect the present invention relates to the use of the polypeptide and variant thereof according to the present invention as anti-microbials in foodstuff, on food processing equipment, in food processing plants, on surfaces coming into contact with foodstuff such as shelves and food deposit areas and in all other situations, where *Clostridium* bacteria can potentially infect food material. Said polypeptides can be used alone or in combination with other anti-microbials like antibiotics. In addition, said polypeptides can be used in animal feed, either alone or in combination with other anti-microbials, for prophylaxis of a disease, e. g. in poultry or pig farming.

In a preferred embodiment the polypeptide according to the present invention is used as medicaments, pharmaceutical compositions, cosmetics, anti-microbials etc.

### Examples

All cloning procedures were performed using standard techniques according to Sambrook et al. (Molecular cloning. A laboratory manual; 2nd ed. Cold Spring Harbor Laboratory Press 1989). Mutations and deletions were also introduced using standard techniques.

### Example 1: Cloning and sequence of phiS9 endolysin and the CBD

Genomic DNA of the *C. perfringens* bacteriophage phiS9 was purified with state of the art techniques and the plyS9 gene as well as the CBD fragments were amplified with standard PCR conditions.

For amplification of the phiS9 endolysin the primer *plyS9-f* having the sequence 5'-CTATCAGGATCCATGCAAAGTAGAAACAATAATAATTTAAAAG-3' (SEQ ID NO:3) and *ply-S9-r* having the sequence 5'-CTATCAGTCGACTTATATTTTCTTAACAAATTCAGCATTAAC-3'(SEQ ID NO:4) were used. *PlyS9-f introduced* a *Bam*H1 restriction site and a start codon, *plyS9-r* introduced a *Sal*I restriction site and a stop codon into the sequence. The amplified gene was cloned into the pQE plasmid (Qiagen; containing the sequence for a 6xHis tag) *via* the *Bam*H1 and *Sal*I restriction sites.

For the amplification of the CBD fragments the forward primers *CBD-S9A-f* having the sequence 5'-ATCAGAGCTCTCAAATAATTTTACTTTAGACAATGC-3' (SEQ ID NO:5), *CBD-S9B-f having* the sequence 5'-ATCAGAGCTCGATATGAATGAGTTCACTAATGG-3' (SEQ ID NO:6), *CBD-S9C-f* having the sequence 5'-ATCAGAGCTCGATTCATGGGTTGGATTCCAATATTC-3' (SEQ ID NO:7) and *CBD-S9D-f* having the sequence 5'-ATCAGAGCTCGATTCATGGGTTGGATTCCAATATTC-3' (SEQ ID NO:8) were used. The reverse primer *ply-S9CBD-r* (SEQ ID NO:9) having the sequence 5'-ATCAGTCGACTTATATTTTCTTAACAAATTCAGC-3' and introducing a *Sal*I restriction site and a stop codon was used for the amplification of all variants. All forward primers introduced a *Sac*I restriction site and a start codon. The amplified genes were cloned into the plasmid pHGFP (Lössner et al., 2002, Mol. Microbiol. 44/2, 335-349) containing *gfp*-mut2 cloned into *Bam*HI-*Sac*I site of pQE30, producing 6xHis-tagged GFP via the *Sac*I and *Sal*I restriction sites. To verify the desired sequences all resulting plasmids were sequenced.

### Example 2: Expression of plyS9 and CBD fragments in E. coli and purification of the recombinant proteins

PlyS9 and the CBD fragments were expressed in *E. coli* XL-1 Blue MRF'. For expression of plyS9 the plasmid pACYC-IRL10 which provides tRNA genes for tRNA^{Arg}, tRNA^{Ile} and TRNA^{Leu} was coexpressed. To achieve better growth of the strain antibiotic concentrations of only 50 µg/ml ampicillin, 15 µg/ml tetracycline and 5 µg/ml chloramphenicol were used. Expression experiments were carried out by inoculating 250 ml LB-PE medium (15 g/l tryptone, 8 g/l yeast extract, 5 g/l NaCl, pH 7.8) containing the above mentioned antibiotics with 5 ml from an overnight culture and shaking at 37 °C (plyS9) or 30°C (CBD fragments) under aeration in 1000 ml flasks. At an optical density at 600 nm (OD₆₀₀) of 0.6 the cultures were induced with 1 mM IPTG and grown for further 4 hours to allow protein production. Samples were taken before induction and 1, 2 and 4 hours after induction for SDS PAGE analysis.. After 4 hours cells were harvested by centrifugation (Beckmann J2-21 with JA-10 rotor, 7000 rpm, 20 min, 10 °C); the pellets were suspended in 8 ml buffer A (50 mM Na-P pH 8.0, 5 mM imidazole, 0.1 % Tween 20, 500 mM NaCl) per 250 ml growth medium. Cells were ruptured by a double passage through a French Pressure Cell Press (SLM Aminco, 1260 PSI, inset: 20k) following one cycle of freezing and thawing at -20 °C to increase the efficiency of cell rupture. Cell debris was removed by centrifugation (Sigma 3K301 with rotor 19776, 20000g, 40 min, 10 °C) and filtration (pore size 0.2 µm) of the supernatant containing the desired proteins. The crude extract was stored on ice for further processing.

Purification of plyS9 and CBD fragments was done using 1 ml Ni-NTA superflow resin (Qiagen) in Micro Bio-Spin Chromatography Columns (Biorad) equilibrated with buffer A. After passage of the crude extract, the column was washed with buffer A (5 column volumes) and a mixture of 10 % buffer B (50 mM Na-P pH 8.0, 250 mM imidazole, 0.1 % Tween 20, 500 mM NaCl) and 90 % buffer A (3 column volumes). Elution of the protein from the column was performed using 100 % buffer B (2 column volumes). Samples from the flow through of crude extract, the washing step with 10 % buffer B and the eluate were taken for SDS PAGE analysis .

Salts and imidazole were removed by dialysis (Spectra/Por, Spectrumlabs, MWCO: 6000-8000) at 4 °C under gentle agitation and 1x buffer exchange after 2 hours. Lowering the salt concentration by dialysis produced substantial loss of protein, so finally only imidazole was removed by using a high salt dialysis buffer (50 mM Na-P pH 8.0, 0.1 % Tween 20, 500 mM NaCl). After dialysis samples were filter sterilized (0.2 µm pore size) and concentrated by centrifugation (Sigma3K30 with 19777 rotor, 8000g, 4 °C in Vivaspin^{™} 4 ml concentrator tubes from Vivascience). The purified proteins were stored at -20 °C in 50 % glycerol.

### Example 3: Lytic activity and substrate specificity ofplyS9

Lytic activity of plyS9 against bacterial cells was determined by measuring the OD₆₀₀ of a cell suspension every 15 seconds during 10 minutes after adding the enzyme (Libra S22 photometer, Biochrom). 6 µg purified plyS9 were brought on the bottom of a half-micro cuvette before filling up to a total volume of 1 ml with substrate cell suspension. Negative controls were done by the same procedure but using a sterile 50:50 mixture of high salt dialysis buffer and glycerol instead of the purified plyS9 solution.

Substrate cells were grown in 5-10 ml of suitable media to an estimated late log or stationary phase. *C*. *perfringens* strains were grown under anaerobic conditions in liquid TYG medium (30 g/l tryptone, 20 g/l yeast extract, 5 g/l glucose, 0.5 g/l L-cysteine, pH 7.2) for approximately 4 hours at 37 °C. *C. tyrobutyricum* strains were handled in the same way. All other Gram positive bacteria were grown under aerobic conditions at 37 °C in TB medium (20 g/l tryptose, 1 g/l glucose, 5 g/l NaCl, 0.005 thiamine HCl pH 7.4) until turbidity was observed. Breeding of strains requiring an elevated CO₂ atmosphere (*Bifidobacterium gallinarum, Lactococcus lactis* and *Leuconostoc mesenteroides*) was performed using GENbox CO₂ generators (BioMérieux) in a gas-proof chamber and MRS broth from Biolife^{™}. Cells were harvested by centrifugation, re-suspended in PBS buffer (50 mM Na-P pH 8.0, 120 mM NaCl) and stored in 20 µl aliquots at -80 °C. Immediately before the preparation of the assays the aliquots were thawn and diluted with 25 mM citrate/phosphate buffer (pH 6.5, 120 mM NaCl). This procedure was necessary because storage of highly concentrated viable *C. perfringens* cells on ice resulted in enhanced autolysis and therefore increased viscosity of the cell suspension after about 15 minutes resulting in low reproducibility of lysis assays.

The endolysin plyS9 showed lytic activity against all 54 *C. perfringens* strains tested.

**Table 1:**

| **Species** | **Strain** | **Lytic activity of plyS9** |
|---|---|---|
| *C. perfringens* | B41 | ++ |
| *C. perfringens* | H1 | ++ |
| *C. perfringens* | H2 | ++ |
| *C. perfringens* | H3 | ++ |
| *C. perfringens* | H4 | ++ |
| *C. perfringens* | H5 | ++ |
| *C. perfringens* | H6 | ++ |
| *C. perfringens* | H7 | ++ |
| *C. perfringens* | H8 | ++ |
| *C. perfringens* | H9 | ++ |
| *C. perfringens* | H10 | ++ |
| *C. perfringens* | H11 | ++ |
| *C. perfringens* | H12 | ++ |
| *C. perfringens* | H13 | ++ |
| *C. perfringens* | H14 | ++ |
| *C. perfringens* | H15 | ++ |
| *C. perfringens* | H16 | ++ |
| *C. perfringens* | H17 | ++ |
| *C. perfringens* | H18 | ++ |
| *C. perfringens* | H19 | ++ |
| *C. perfringens* | H20 | + |
| *C. perfringens* | H21 | ++ |
| *C. perfringens* | H22 | ++ |
| *C. perfringens* | ATCC3626 | ++ |
| *C. perfringens* | ATCC3628 | ++ |
| *C. perfringens* | DSM 756T | ++ |
| *C. perfringens* | DSM 798 | ++ |
| *C. perfringens* | DSM 2943 | ++ |
| *C. perfringens* | LMU 756 | ++ |
| *C. perfringens* | LMU 1653 | ++ |
| *C. perfringens* | LMU 1677 | ++ |
| *C. perfringens* | LMU 1678 | ++ |
| *C. perfringens* | LMU 1679 | ++ |
| *C. perfringens* | LMU 1695 | ++ |
| *C. perfringens* | LMU 1796 | ++ |
| *C. perfringens* | LMU 1798 | ++ |
| *C. perfringens* | LMU 1799 | ++ |
| *C. perfringens* | LMU1802 | ++ |
| *C. perfringens* | NCTC 528 | ++ |
| *C. perfringens* | NCTC 3110 | ++ |
| *C. perfringens* | NCTC 6719 | ++ |
| *C. perfringens* | NCTC6785 | ++ |
| *C. perfringens* | NCTC 8081 | ++ |
| *C. perfringens* | NCTC 8346 | ++ |
| *C. perfringens* | NCTC 8533 | ++ |
| *C. perfringens* | NCTC 10240 | ++ |
| *C. perfringens* | NCTC 10612 | ++ |
| *C. perfringens* | NCTC 10614 | ++ |
| *C. perfringens* | NCTC 10719 | ++ |
| *C. perfringens* | NCTC 11144 | ++ |
| *C. perfringens* | 025 | ++ |
| *C. perfringens* | S9 | ++ |
| *C. perfringens* | S 13 | ++ |
| *C. perfringens* | U12 | ++ |

| | | |
|---|---|---|
| **Interpretation of lytic activities:** ++ decrease of OD₆₀₀ more than 50% within 10 minutes + decrease of OD₆₀₀ more than 10% (+) decrease of OD₆₀₀ more than 4% and a typical lysis curve must be observable - no significant decrease of OD₆₀₀ | | |

Furthermore some *C*. *tyrobutyricum* strains and *Bacillus megaterium* DSM 90 were lysed by plyS9.

**Table 2:**

| **Species** | **Strain** | **Lytic activity of plyS9** |
|---|---|---|
| *C. tyrobutyricum* | DSM 663 | (+) |
| *C. tyrobutyricum* | DSM 664 | (+) |
| *C. tyrobutyricum* | DSM 1460 | - |
| *C. tyrobutyricum* | DSM 2637 | - |
| *C. tyrobutyricum* | FAM 1353 | - |
| *C. tyrobutyricum* | FAM 1519 | + |
| *C. tyrobutyricum* | FAM 1520 | - |
| *C. tyrobutyricum* | FAM 1528 | - |
| *C. tyrobutyricum* | FAM 1555 | - |
| *C. tyrobutyricum* | FAM 1556 | - |
| *C. tyrobutyricum* | FAM 1557 | - |
| *C. tyrobutyricum* | FAM 1558 | (+) |
| *C. tyrobutyricum* | FAM 1559 | - |
| *C. tyrobutyricum* | FAM 1617 | (+) |
| *Bacillus cereus* | DSM 31 | - |
| *Bacillus cereus* | DSM 307 | - |
| *Bacillus megaterium* | DSM 90 | + |
| *acillus sphaericus* | DSM 395 | - |
| *Bacillus subtilis* | DSM 168 | - |
| *Bacillus subtilis* | DSM 675 | - |
| *Bacillus thuringiensis* | DSM 4412 | - |
| *Bacillus thuringiensis* | DSM 4421 | - |
| *Bifidobacterium gallinarum* | DSM 20760T | - |
| *Enterococcus faecalis* | ATCC 19433 | - |
| *Enterococcus facium* | DSM 20477 | - |
| *Enterococcus hirae* | DSM 20160 | - |
| *Lactococcus lactis* | Bu2 | - |
| *Lactococcus lactis spp. Cremoris* | AC | - |
| *Leuconostoc mesenteroides* | 37-6 | - |
| *Staphylococcus aureus* | DSM 1104 | - |
| *Staphylococcus aureus* | DSM2569 | - |
| *Streptococcus thermophilus* | 62 | - |
| *Staphylococcus equorum* | SB2 | - |

| | | |
|---|---|---|
| **Interpretation of lytic activities:** ++ decrease of OD₆₀₀ more than 50% within 10 minutes + decrease of OD₆₀₀ more than 10% (+) decrease of OD₆₀₀ more than 4% and a typical lysis curve must be observable - no significant decrease of OD₆₀₀ | | |

### Example 4: Influence of pH, salt and metal ions on lytic activity of plyS9

To test the influence of pH, salt concentrations and divalent metal-ions on plyS9 activity viable *C. perfringens* S 13 cells were used as substrate. For optimal comparison of the lysis curves 0.5 µg plyS9 was applied per 1 ml assay volume.

For the pH optimization assays, cells from frozen batches were suspended and thawn in 25 mM citrate/phosphate buffers (pH 4.5 - 6.5; 120 mM NaCl) or in 25 mM Tris/phosphate buffers (pH 5.5 - 9.0; 120 mM NaCl) immediately before mixing the substrate cell suspension with the enzyme. plyS9 activity was found to be highest at pH between 6.0 and 6.5 corresponding to its isoelectric point of pH 6.17. At pH values higher than 8.0 activity decreased considerably and at pH 4.0 or below absolutely no lytic activity could be observed. Sodium chloride influence was tested using 20 mM MOPS buffer pH 7.0 containing different sodium chloride concentrations. Optimal sodium chloride concentration was found to be 300 mM.

The influence of divalent metal-ions was tested as follows: A volume of 100 µl metal-ion solution (100 mM CaCl₂, CoCl₆*6H₂O, CuCl₂*2H₂O, MgCl₂, MnSO₄*H₂O, NiCl₂*2H₂O, ZnCl₂, FeCl₂*H₂O [prepared and stored under anaerobic conditions], in heat sterilized Milli-Q water) or 100 µl of 1:10 diluted metal-ion solution was poured in a half-micro cuvette. The enzyme (0.5 µg) was dropped on the wall of the cuvette above the metal-ion solution. Substrate cells, diluted with 20 mM MOPS pH 7.0, 300 mM NaCl, were used to fill up the assays to 1 ml. The final metal-ion concentrations were 10 mM or 1 mM, respectively. Calcium had the greatest enhancing influence on plyS9 activity, followed by magnesium. The higher the respective concentration was, the higher the enzymatic activity was. Manganese had a positive influence when its concentration was low (1 mM), but this effect disappeared when manganese concentration was 10fold higher. Cobalt lowered the lytic activity by about 20 % when its concentration in the assay was only 1 mM. A 10 mM cobalt concentration inactivated the enzyme. This could also be observed for any addition of iron, copper, nickel and zinc.

### Example 5: Binding properties of CBDS9 C

For comparison of the binding properties of the different variants an equal amount of protein molecules was added to each binding assay. The calculated protein volumes were diluted with PBST buffer to a final volume of 50 µl and mixed with the target cells. After incubation of 15 minutes at room temperature cells were washed twice with 500 µl PBST buffer to remove excess and unbound fluorescent protein and finally they were re-suspended in 50 µl PBST buffer. Binding assay pictures of CBDS9 fusion proteins on all tested *C. perfringens* strains were taken with a Zeiss Axioplan microscope (100x ocular) using a Zeiss filter-set (excitation: BP 450-49 nm, beamsplitter: FT 510 nm, emission: LP 520 nm). Assays with CBD3626 and all assays with *C. tyrobutyricum* strains were beheld under a Leica DMI 4000 CSQ microscope (Epi-fluorescence, 63x ocular); Kretzer et al., 2007, Appl. and Environ. Microbiol. 73/06, 1992-2000.

All four length variants of CBDS9 label the reference strains *C. perfringens* S9 and S13. The variant CBDS9_C was chosen for further binding assays.

**Table 3:**

| | | **Binding of** | |
|---|---|---|---|
| **Species** | **Strain** | **CBDS9_C** | **CBD3626** |
| *C. perfringens* | B41 | + | + |
| *C. perfringens* | H1 | + | + |
| *C. perfringens* | H2 | ++ | ++ |
| *C. perfringens* | H3 | ++ | ++ |
| *C. perfringens* | H4 | ++ | + |
| *C. perfringens* | H5 | ++ | + |
| *C. perfringens* | H6 | + | + |
| *C. perfringens* | H7 | ++ | ++ |
| *C. perfringens* | H8 | ++ | + |
| *C. perfringens* | H9 | ++ | ++ |
| *C. perfringens* | H10 | ++ | + |
| *C. perfringens* | H11 | ++ | ++ |
| *C. perfringens* | H12 | ++ | + |
| *C. perfringens* | H13 | ++ | ++ |
| *C. perfringens* | H 14 | ++ | ++ |
| *C. perfringens* | H15 | + | + |
| *C. perfringens* | H16 | + | + |
| *C. perfringens* | H 17 | ++ | ++ |
| *C. perfringens* | H18 | + | ++ |
| *C. perfringens* | H19 | ++ | ++ |
| *C. perfringens* | H20 | ++ | ++ |
| *C. perfringens* | H21 | + | + |
| *C. perfringens* | H22 | ++ | + |
| *C. perfringens* | ATCC3626 | ++ | ++ |
| *C. perfringens* | ATCC3628 | ++ | ++ |
| *C. perfringens* | DSM 756T | ++ | ++ |
| *C. perfringens* | DSM 798 | ++ | ++ |
| *C. perfringens* | DSM 2943 | + | ++ |
| *C. perfringens* | LMU 756 | ++ | ++ |
| *C. perfringens* | LMU 1653 | ++ | + |
| *C. perfringens* | LMU 1677 | ++ | ++ |
| *C. perfringens* | LMU 1678 | ++ | ++ |
| *C. perfringens* | LMU 1679 | ++ | ++ |
| *C. perfringens* | LMU 1695 | ++ | + |
| *C. perfringens* | LMU 1796 | ++ | ++ |
| *C. perfringens* | LMU 1798 | ++ | + |
| *C. perfringens* | LMU 1799 | ++ | ++ |
| *C. perfringens* | LMU1802 | ++ | + |
| *C. perfringens* | NCTC 528 | + | + |
| *C. perfringens* | NCTC 3110 | ++ | ++ |
| *C. perfringens* | NCTC 6719 | ++ | ++ |
| *C. perfringens* | NCTC6785 | ++ | ++ |
| *C. perfringens* | NCTC 8081 | ++ | ++ |
| *C. perfringens* | NCTC 8346 | ++ | ++ |
| *C. perfringens* | NCTC 8533 | ++ | ++ |
| *C. perfringens* | NCTC 10240 | ++ | ++ |
| *C. perfringens* | NCTC 10612 | + | + |
| *C. perfringens* | NCTC 10614 | ++ | ++ |
| *C. perfringens* | NCTC 10719 | ++ | + |
| *C. perfringens* | NCTC 11144 | ++ | ++ |
| *C. perfringens* | 025 | ++ | ++ |
| *C. perfringens* | S9 | ++ | + |
| *C. perfringens* | S13 | ++ | + |
| *C. perfringens* | U12 | ++ | ++ |

| | | | |
|---|---|---|---|
| **Interpretation of binding properties:** ++ cells displayed an intensive green fluorescence, either only at poles and septae or the whole cell (strong binding) + cells could easily be seen with less intensitiy (weak binding) (+) cells disappeared quickly after excitation (very weak binding) - no cells could be seen (no binding) | | | |

**Table 4:**

| | | **Binding of** | |
|---|---|---|---|
| **Species** | **Strain** | **CBDS9_C** | **CBD3626** |
| *C. tyrobutyricum* | DSM 663 | (+) | + |
| *C. tyrobutyricum* | DSM 664 | (+) | + |
| *C. tyrobutyricum* | DSM 1460 | - | + |
| *C. tyrobutyricum* | DSM 2637 | (+) | (+) |
| *C. tyrobutyricum* | FAM 1353 | (+) | (+) |
| *C. tyrobutyricum* | FAM 1519 | (+) | (+) |
| *C. tyrobutyricum* | FAM 1520 | - | (+) |
| *C. tyrobutyricum* | FAM 1528 | - | - |
| *C. tyrobutyricum* | FAM 1555 | - | + |
| *C. tyrobutyricum* | FAM 1556 | - | + |
| *C. tyrobutyricum* | FAM 1557 | - | (+) |
| *C. tyrobutyricum* | FAM 1558 | - | - |
| *C. tyrobutyricum* | FAM 1559 | - | - |
| *C. tyrobutyricum* | FAM 1617 | + | (+) |
| *Bacillus cereus* | DSM 31 | - | - |
| *Bacillus cereus* | DSM 307 | - | - |
| *Bacillus megaterium* | DSM 90 | + | - |
| *Bacillus sphaericus* | DSM 395 | - | + |
| *Bacillus subtilis* | DSM 168 | - | - |
| *Bacillus subtilis* | DSM 675 | - | - |
| *Bacillus thuringiensis* | DSM 4412 | - | - |
| *Bacillus thuringiensis* | DSM 4421 | - | ++ |
| *Bifidobacterium gallinarum* | DSM 20760T | - | - |
| *Enterococcus faecalis* | ATCC 19433 | - | - |
| *Enterococcus facium* | DSM 20477 | - | - |
| *Enterococcus hirae* | DSM 20160 | - | - |
| *Lactococcus lactis* | Bu2 | - | - |
| *Lactococcus lactis spp. Cremoris* | AC1 | - | - |
| *Leuconostoc mesenteroides* | 37-6 | - | - |
| *Staphylococcus aureus* | DSM 1104 | - | - |
| *Staphylococcus aureus* | DSM2569 | - | - |
| *Streptococcus thermophilus* | 62 | - | - |
| *Staphylococcus equorum* | SB2 | - | - |

| | | | |
|---|---|---|---|
| **Interpretation of binding properties:** ++ cells displayed an intensive green fluorescence, either only at poles and septae or the whole cell (strong binding) + cells could easily be seen with less intensitiy (weak binding) (+) cells disappeared quickly after excitation (very weak binding) - no cells could be seen (no binding) | | | |

## Claims

1. A polypeptide having the amino acid sequence as depicted in SEQ ID NO: or a fragment thereof

2. Polypeptide according to claim 1, wherein the fragment has the biological activity of binding the cell wall of *C. perfringens.*

3. Polypeptide according to claim 1 or 2, wherein the fragment comprises amino acid residues beginning at an amino acid residue in the range of 170 and 203 and ending at the amino acid residue 342 according to the amino acid sequence depicted in SEQ m NO:1

4. Polypeptide according to any one of claims 1-3 as depicted in SEQ ID NO:10-13.

5. Polypeptide according to any one of the preceding claims, wherein the polypeptide comprises a biotin residue, an HA-tag, His-tag, Strep-tag, Myc-tag, Js-tag or GST-tag.

6. A nucleic acid molecule comprising a nucleotide sequence encoding a polypeptide according to any one of claims 1 to 5.

7. A vector comprising the nucleic acid according to claim 6.

8. A host transformed with the nucleic acid molecule according to claim 6 or a vector according to claim 7.

9. A composition comprising a polypeptide according to any one of claims 1 to 5.

10. A composition according to claim 9, wherein the composition is a pharmaceutical composition and further comprises a pharmaceutically acceptable diluent, excipient or carrier.

11. A polypeptide according to any one of claims 1 to 5 for use as a human or animal therapeutic or diagnostic agent.

12. A polypeptide according to any one of claims 1 to 5 for use as a human or animal therapeutic or diagnostic agent for treatment, prevention or diagnosis of a disorder, disease or condition associated with pathogenic *Clostridium* species, preferably with *Clostridium perfringens.*

13. Use of the polypeptide according to anyone of claims 1 to 5 for the treatment, prevention or diagnosis of *Clostridium* contamination of foodstuff, of food processing equipment, of food processing plants, or of surfaces coming into contact with foodstuff.


SEQUENCE LISTING

<110>  Profos AG

<120>  New enzymatic active enzyme against Clostridium

<130>  PRO-035 EP

<140>  unknown
<141>  2008-07-07

<160>  14

<170>  PatentIn version 3.3

<210>  1
<211>  342
<212>  PRT
<213>  Bacteriophage S9

<400>  1

Met Gln Ser Arg Asn Asn Asn Asn Leu Lys Gly Ile Asp Val Ser Asn
1               5                   10                  15
Trp Lys Gly Asn Ile Asn Phe Gln Ser Val Lys Asn Asp Gly Val Glu
            20                  25                  30
Val Val Tyr Ile Lys Ala Thr Glu Gly Asn Tyr Phe Lys Asp Lys Tyr
        35                  40                  45
Ala Lys Gln Asn Tyr Glu Arg Ala Lys Glu Gln Gly Leu Arg Val Gly
    50                  55                  60
Phe Tyr His Phe Phe Arg Ala Asn Lys Gly Ala Lys Asp Gln Ala Asn
65                  70                  75                  80
Phe Phe Val Asn Tyr Leu Asn Glu Ile Gly Ala Val Asn Tyr Asp Cys
                85                  90                  95
Lys Leu Ala Leu Asp Ile Glu Thr Thr Glu Gly Val Gly Ala Arg Asp
            100                 105                 110
Leu Thr Ser Met Cys Ile Glu Phe Leu Glu Glu Val Lys Arg Leu Thr
        115                 120                 125
Gly Lys Glu Val Val Val Tyr Thr Tyr Thr Ser Phe Ala Asn Asn Asn
    130                 135                 140
Leu Asp Ser Arg Leu Gly Asn Tyr Pro Val Trp Ile Ala His Tyr Gly
145                 150                 155                 160
Val Asn Thr Pro Gly Ala Asn Asn Ile Trp Asp Ser Trp Val Gly Phe
                165                 170                 175
Gln Tyr Ser Glu Asn Gly Ser Val Ala Gly Val Ser Gly Gly Cys Asp
            180                 185                 190
Met Asn Glu Phe Thr Asn Gly Ile Phe Ile Asp Ser Asn Asn Phe Thr
        195                 200                 205
Leu Asp Asn Ala Thr Thr Lys Asn Val Ser Ile Lys Leu Asn Ile Arg
    210                 215                 220
Ala Lys Gly Thr Thr Asn Ser Lys Val Ile Gly Ser Ile Ser Ala Gly
225                 230                 235                 240
Glu Thr Phe Lys Ile Lys Trp Val Asp Glu Asp Tyr Leu Gly Trp Tyr
                245                 250                 255
Tyr Val Glu Tyr Asn Gly Ile Val Gly Tyr Val Asn Ala Asp Tyr Val
            260                 265                 270
Glu Lys Leu Gln Met Ala Thr Thr His Asn Val Ser Thr Phe Leu Asn
        275                 280                 285
Val Arg Glu Glu Gly Ser Leu Asn Ser Arg Ile Val Asp Lys Ile Asn
    290                 295                 300
Ala Gly Asp Ile Phe Arg Ile Asp Trp Val Asp Glu Asp Phe Ile Gly
305                 310                 315                 320
Trp Tyr Arg Val Thr Thr Lys Asn Gly Lys Val Gly Phe Val Asn Ala

325                 330                 335
Glu Phe Val Lys Lys Ile
            340

<210>  2
<211>  1029
<212>  DNA
<213>  Bakteriophagen S9

<400>  2
atgcaaagta gaaacaataa taatttaaaa ggaattgatg tatcaaactg gaaaggaaat      60
ataaattttc aaagtgtaaa aaatgatggt gtagaagtag tttatattaa agctacagaa     120
ggtaattact ttaaggataa atatgctaaa caaaattatg agagagcgaa agagcaagga     180
ttaagagtag gattttatca tttctttaga gctaataaag gagccaagga tcaagcaaat     240
tttttcgtaa attatttaaa tgaaatagga gcagttaatt atgattgtaa attagcttta     300
gatatagaaa ctactgaagg tgtaggagca agagatttaa cttctatgtg tatagaattc     360
ttagaagagg taaaaagact tactggaaaa gaagttgttg tatatacata tacaagcttt     420
gcaaataaca acttagatag tagactaggt aattatccag tttggattgc acattatggg     480
gtaaacactc caggagccaa taacatatgg gattcatggg ttggattcca atattcagag     540
aatggaagtg tagctggtgt aagtggtgga tgtgatatga atgagttcac taatggaata     600
ttcattgatt caaataattt tactttagac aatgctacta ctaaaaatgt aagtattaaa     660
ttaaatataa gggctaaagg aactactaat tctaaagtaa ttggctcaat atcagctggc     720
gaaacattta aaataaaatg ggttgatgaa gattatcttg gttggtatta cgttgagtat     780
aatggaatag ttggctatgt aaatgccgat tatgtagaaa agctacaaat ggctactact     840
cataatgtaa gtactttttt aaatgtaaga gaagaaggat cattgaattc tagaatagta     900
gataagataa atgcaggtga tatttttaga attgattggg tagatgaaga tttcattgga     960
tggtatagag taacaactaa aaatggtaaa gtaggctttg ttaatgctga atttgttaag    1020
aaaatataa                                                            1029

<210>  3
<211>  43
<212>  DNA
<213>  artificial sequence

<220>
<223>  primer plyS9-f

<400>  3
ctatcaggat ccatgcaaag tagaaacaat aataatttaa aag                        43

<210>  4
<211>  42
<212>  DNA
<213>  artificial sequence

<220>
<223>  primer ply-S9-r

<400>  4
ctatcagtcg acttatattt tcttaacaaa ttcagcatta ac                         42

<210>  5
<211>  36
<212>  DNA
<213>  artificial sequence

<220>
<223>  primer CBD-S9A-f

<400> 5
atcagagctc tcaaataatt ttactttaga caatgc 36

<210> 6
<211> 33
<212> DNA
<213> artificial sequence

<220>
<223> primer CBD-S9B-f

<400> 6
atcagagctc gatatgaatg agttcactaa tgg 33

<210> 7
<211> 36
<212> DNA
<213> artificial sequence

<220>
<223> primer CBD-S9C-f

<400> 7
atcagagctc gattcatggg ttggattcca atattc 36

<210> 8
<211> 36
<212> DNA
<213> artificial sequence

<220>
<223> primer CBD-S9D-f

<400> 8
atcagagctc gattcatggg ttggattcca atattc 36

<210> 9
<211> 34
<212> DNA
<213> artificial sequence

<220>
<223> primer ply-S9CBD-r

<400> 9
atcagtcgac ttatattttc ttaacaaatt cagc 34

<210> 10
<211> 173
<212> PRT
<213> artificial sequence

<220>
<223> CBD von plyS9

<400> 10

Trp Asp Ser Trp Val Gly Phe Gln Tyr Ser Glu Asn Gly Ser Val Ala

1               5                   10                  15
Gly Val Ser Gly Gly Cys Asp Met Asn Glu Phe Thr Asn Gly Ile Phe
            20                  25                  30
Ile Asp Ser Asn Asn Phe Thr Leu Asp Asn Ala Thr Thr Lys Asn Val
        35                  40                  45
Ser Ile Lys Leu Asn Ile Arg Ala Lys Gly Thr Thr Asn Ser Lys Val
    50                  55                  60
Ile Gly Ser Ile Ser Ala Gly Glu Thr Phe Lys Ile Lys Trp Val Asp
65                  70                  75                  80
Glu Asp Tyr Leu Gly Trp Tyr Tyr Val Glu Tyr Asn Gly Ile Val Gly
                85                  90                  95
Tyr Val Asn Ala Asp Tyr Val Glu Lys Leu Gln Met Ala Thr Thr His
            100                 105                 110
Asn Val Ser Thr Phe Leu Asn Val Arg Glu Glu Gly Ser Leu Asn Ser
        115                 120                 125
Arg Ile Val Asp Lys Ile Asn Ala Gly Asp Ile Phe Arg Ile Asp Trp
    130                 135                 140
Val Asp Glu Asp Phe Ile Gly Trp Tyr Arg Val Thr Thr Lys Asn Gly
145                 150                 155                 160
Lys Val Gly Phe Val Asn Ala Glu Phe Val Lys Lys Ile
                165                 170

<210>  11
<211>  162
<212>  PRT
<213>  artificial sequence

<220>
<223>  CBD von plyS9

<400>  11

Asn Gly Ser Val Ala Gly Val Ser Gly Gly Cys Asp Met Asn Glu Phe
1               5                   10                  15
Thr Asn Gly Ile Phe Ile Asp Ser Asn Asn Phe Thr Leu Asp Asn Ala
            20                  25                  30
Thr Thr Lys Asn Val Ser Ile Lys Leu Asn Ile Arg Ala Lys Gly Thr
        35                  40                  45
Thr Asn Ser Lys Val Ile Gly Ser Ile Ser Ala Gly Glu Thr Phe Lys
    50                  55                  60
Ile Lys Trp Val Asp Glu Asp Tyr Leu Gly Trp Tyr Tyr Val Glu Tyr
65                  70                  75                  80
Asn Gly Ile Val Gly Tyr Val Asn Ala Asp Tyr Val Glu Lys Leu Gln
                85                  90                  95
Met Ala Thr Thr His Asn Val Ser Thr Phe Leu Asn Val Arg Glu Glu
            100                 105                 110
Gly Ser Leu Asn Ser Arg Ile Val Asp Lys Ile Asn Ala Gly Asp Ile
        115                 120                 125
Phe Arg Ile Asp Trp Val Asp Glu Asp Phe Ile Gly Trp Tyr Arg Val
    130                 135                 140
Thr Thr Lys Asn Gly Lys Val Gly Phe Val Asn Ala Glu Phe Val Lys
145                 150                 155                 160
Lys Ile

<210>  12
<211>  151
<212>  PRT
<213>  artificial sequence

<220>

<223>  CBD von plyS9

<400>  12

Asp Met Asn Glu Phe Thr Asn Gly Ile Phe Ile Asp Ser Asn Asn Phe
1               5                   10                  15
Thr Leu Asp Asn Ala Thr Thr Lys Asn Val Ser Ile Lys Leu Asn Ile
            20                  25                  30
Arg Ala Lys Gly Thr Thr Asn Ser Lys Val Ile Gly Ser Ile Ser Ala
        35                  40                  45
Gly Glu Thr Phe Lys Ile Lys Trp Val Asp Glu Asp Tyr Leu Gly Trp
    50                  55                  60
Tyr Tyr Val Glu Tyr Asn Gly Ile Val Gly Tyr Val Asn Ala Asp Tyr
65                  70                  75                  80
Val Glu Lys Leu Gln Met Ala Thr Thr His Asn Val Ser Thr Phe Leu
                85                  90                  95
Asn Val Arg Glu Glu Gly Ser Leu Asn Ser Arg Ile Val Asp Lys Ile
            100                 105                 110
Asn Ala Gly Asp Ile Phe Arg Ile Asp Trp Val Asp Glu Asp Phe Ile
        115                 120                 125
Gly Trp Tyr Arg Val Thr Thr Lys Asn Gly Lys Val Gly Phe Val Asn
    130                 135                 140
Ala Glu Phe Val Lys Lys Ile
145                 150


<210>  13
<211>  140
<212>  PRT
<213>  artificial sequence

<220>
<223>  CBD von plyS9

<400>  13

Asp Ser Asn Asn Phe Thr Leu Asp Asn Ala Thr Thr Lys Asn Val Ser
1               5                   10                  15
Ile Lys Leu Asn Ile Arg Ala Lys Gly Thr Thr Asn Ser Lys Val Ile
            20                  25                  30
Gly Ser Ile Ser Ala Gly Glu Thr Phe Lys Ile Lys Trp Val Asp Glu
        35                  40                  45
Asp Tyr Leu Gly Trp Tyr Tyr Val Glu Tyr Asn Gly Ile Val Gly Tyr
    50                  55                  60
Val Asn Ala Asp Tyr Val Glu Lys Leu Gln Met Ala Thr Thr His Asn
65                  70                  75                  80
Val Ser Thr Phe Leu Asn Val Arg Glu Glu Gly Ser Leu Asn Ser Arg
                85                  90                  95
Ile Val Asp Lys Ile Asn Ala Gly Asp Ile Phe Arg Ile Asp Trp Val
            100                 105                 110
Asp Glu Asp Phe Ile Gly Trp Tyr Arg Val Thr Thr Lys Asn Gly Lys
        115                 120                 125
Val Gly Phe Val Asn Ala Glu Phe Val Lys Lys Ile
    130                 135                 140


<210>  14
<211>  66
<212>  PRT
<213>  artificial sequence

<220>
<223>  JS-Tag

<400>  14

Val Thr Ala Pro Leu Ala Gly Thr Ile Trp Lys Val Leu Ala Ser Glu
1               5                   10                  15
Gly Gln Thr Val Ala Ala Gly Glu Val Leu Leu Ile Leu Glu Ala Met
            20                  25                  30
Lys Met Glu Thr Glu Ile Arg Ala Ala Gln Ala Gly Thr Val Arg Gly
        35                  40                  45
Ile Ala Val Lys Ala Gly Asp Ala Val Ala Val Gly Glu Thr Leu Met
    50                  55                  60
Thr Leu
65